# EUROPÄISCHE PATENTSCHRIFT

(11) **EP 2 729 198 B1**
(45) Veröffentlichungstag und Bekanntmachung des Hinweises auf die Patenterteilung: **01.02.2017**
(21) Anmeldenummer: 12740081.0
(22) Anmeldetag: 29.06.2012
(51) Int. Cl.: A61M 1/16

(54) **DIALYSEVERFAHREN ZUR ENTFERNUNG PROTEINGEBUNDENER TOXINE AUS DEM BLUT VON PATIENTEN UNTER EINSATZ HOCHFREQUENTER, ELEKTROMAGNETISCHER FELDER**
DIALYSIS METHOD FOR REMOVING PROTEIN-BONDED TOXINS FROM THE BLOOD OF PATIENTS USING HIGH-FREQUENCY ELECTROMAGNETIC FIELDS
PROCÉDÉ DE DIALYSE POUR EXTRAIRE, DU SANG DE PATIENTS, DES TOXINES LIÉES AUX PROTÉINES AU MOYEN DE CHAMPS ÉLECTROMAGNÉTIQUES HAUTE FRÉQUENCE

(30) Priorität: 05.07.2011 DE 102011078695
(43) Veröffentlichungstag der Anmeldung: 14.05.2014
(73) Patentinhaber: Baxter International Inc., Deerfield, IL 60015 (US); Baxter Healthcare SA, 8152 Glattpark (Opfikon) (CH)
(72) Erfinder: JANKOWSKI, Joachim, 14532 Stahnsdorf (DE); ZIDEK, Walter, 14167 Berlin (DE); BRETTSCHNEIDER, Falko, 14052 Berlin (DE); JANKOWSKI, Vera, 14532 Stahnsdorf (DE)
(74) Vertreter: Gulde & Partner
(86) Internationale Anmeldenummer: PCT/EP2012/062657
(87) Internationale Veröffentlichungsnummer: WO 2013/004604

(56) Entgegenhaltungen:
- DE-A1-102008 050 849
- FR-A5- 2 087 416

## Beschreibung

Primäre Aufgabe der Nieren ist die Ausscheidung harnpflichtiger Substanzen, den sogenannten Urämie-Toxinen. Nieren chronisch-niereninsuffizienter Patienten können diese Aufgaben nicht mehr erfüllen, was im unbehandelten Zustand rasch zur Vergiftung des Patienten und damit zum Tod führt. Die Dialyse ist das Instrument der Wahl zur Linderung der akuten und chronischen Erkrankung, und zur zeitlichen Überbrückung bis ein geeignetes Spenderorgan zur Verfügung steht. Die Dialyse beruht auf dem Prinzip des Stoffaustauschs durch Filtration bzw. Diffusion. Dabei wirken die derzeit verwendeten Membranen als reine Filter- und/oder Diffusionsmembranen und sorgen dafür, dass dem zu reinigenden Blut Stoffe entzogen werden, die eine bestimmte Größe nicht überschreiten. Derzeit gebräuchliche Dialysemembranen haben eine Ausschlussgrenze von beispielsweise 14.000-17.000 Da. Durch die derzeit angewandten Dialysetechniken ist aber im Regelfall eine vollständige Abtrennung der Urämie-Toxine nicht möglich, da ein Teil der harnpflichtigen Substanzen proteingebundenen vorliegen. Hierbei handelt sich unter anderem um niedermolekulare, aromatische Substanze. Urämie-Toxine, die in der Regel proteingebunden vorliegen können, sind beispielsweise Phenylessigsäure, p-Hydroxyhippursäure und Indoxylsulfat. In der Folge akkumulieren die betreffenden Substanzen im Organismus des Patienten und bedingen dort Folgeerkrankungen der akuten und chronischen Niereninsuffizienz. Bei chronisch niereninsuffizienten Patienten treten dadurch bedingt zunehmend Folgeerkrankungen wie Herzkreislauferkrankungen, und daraus resultierend eine erhöhte Sterblichkeitsrate auf. Die Dialyse beschleunigt z. B. die Entstehung von Arteriosklerose. Gefäßerkrankungen wie Arteriosklerose verursachen die häufigsten Todesfälle bei dieser Patientengruppe.

Ein Grund dafür sind niedermolekulare, hydrophobe und aromatische Urämie-Toxine. Aromatische, hydrophobe Urämie-Toxine weisen eine geringe Wasserlöslichkeit auf. Daher kommt es zwischen diesen Substanzen und Plasmaproteinen meist zur Ausbildung adsorptiver Effekte. Diese adsorptiven Effekte gehen auf unterschiedliche Wechselwirkungen zurück. Hierbei sind vor allem Wasserstoffbrückenbindungen, Ionenbindungen und Dipol-Dipol-Wechselwirkungen (van-der-Waals-Kräfte) zu nennen. Durch die Bindung an Plasmaproteine, wie z. B. Albumin, kann das effektive Molekulargewicht der harnpflichtigen Substanzen derart erhöht werden, dass effektive Molekulargewichte von deutlich größer als 17.000 Da erreicht werden. Das resultierende Molekulargewicht der proteingebundenen Urämie-Toxine liegt dadurch oberhalb der Ausschlussgrenze der verwendeten Dialysemembranen und diese können somit bei der Dialyse nicht effektiv entfernt werden.

Durch die Dialyse kann folglich nur der nicht-proteingebundene Anteil des betreffenden Urämie-Toxins abgetrennt werden. Der proteingebundene Anteil (bis zu 95 % der Gesamtmenge des Urämie-Toxins) wird dadurch nicht grundlegend verändert. Aufgrund des Gleichgewichts gemäß des Massenwirkungsgesetz zwischen proteingebundenen und nicht-proteingebundenen Urämie-Toxinen werden unmittelbar nach der Dialyse wieder im Wesentlichen die initialen, nicht-proteingebundene Urämie-Toxinkonzentrationen im Plasma chronisch-niereninsuffizienter Patienten erreicht. Da die pathophysiologischen und pathochemischen Wirkungen im Besonderen von nicht-proteingebundenen Urämie-Toxinen bedingt werden, wird durch diese Gleichgewichtseinstellung ein für die Patienten fataler Prozess initiiert. Dieser *circulus vitiosus* ist Ursache für die vielfältigen pathologischen Erscheinungsformen der chronischen Niereninsuffizienz. Bis zum heutigen Tag sind keine konventionellen Methoden verfügbar, durch die auch protein-gebundene Urämie-Toxine während der Dialyse effektiv aus dem zu reinigenden Blut entfernt werden können.

Der vorliegenden Erfindung liegt die Aufgabe zugrunde mindestens einen Nachteil des geschilderten Standes der Technik zu vermindern oder zu vermeiden. Insbesondere ist es eine Aufgabe der Erfindung Mittel und Wege zur Verfügung zu stellen, mit denen protein-gebundene Urämie-Toxine effektiv aus dem Blut von Dialysepatienten entfernt werden können.

Die Aufgabe wird gelöst durch die Verwendung eines hochfrequenten, elektromagnetischen Feldes in einem Verfahren zur Dialyse mittels eines Dialysators zum Stoffaustausch, insbesondere zur Hämodialyse oder Hämodiafiltration, dadurch gekennzeichnet, dass das zu reinigende Blut vor und/oder während des Kontaktes mit dem Dialysator einem hochfrequenten, elektromagnetischen Feld mit einer Frequenz von 0,0001 GHz bis 1 GHz ausgesetzt wird.

Der Erfindung liegt die Erkenntnis zugrunde, dass die Bindungen zwischen Urämie-Toxinen und Plasmaproteinen in der Regel keine *"echten"* chemischen (kovalenten) Bindungen sind, sondern es sich um reversible Bindungen handelt. Diese Bindungen beruhen im Wesentlichen auf den elektrostatischen Eigenschaften und Wechselwirkungen der beteiligten Moleküle. Es wurde gefunden, dass die Stärke dieser Bindungen oder Wechselwirkungen erfindungsgemäß durch die Einwirkung von hochfrequenten elektromagnetischen Feldern herabgesetzt werden kann. Durch die Verwendung hochfrequenter elektromagnetischer Felder während der Dialyse kann der Anteil proteingebundener Urämie-Toxine deutlich reduziert werden. Im Rahmen der im klinischen Alltag durchgeführten Dialyse kann durch zusätzliche Anwendung hochfrequenter elektromagnetischer Felder die Freisetzungsrate proteingebundener Urämie-Toxine aus der Proteinbindung erhöht werden. Dadurch wird während der Dialyse eine verbesserte Abtrennung dieser Substanzen aus dem Blut des Patienten erreicht. Die betreffenden Urämie-Toxine können somit vermehrt und effektiver dialysiert werden.

Im Rahmen der erfindungsgemäßen Verwendung wird ein Dialysator zum Stoffaustausch verwendet. Aufgabe des Dialysators ist es, aus dem zu reinigenden Blut Urämie-Toxine möglichst effektiv zu entfernen. Im Dialysator sind zu reinigendes Blut und eine Flüssigkeit, gegen die dialysiert werden soll, das sog. Dialysat, durch eine semipermeable Membran voneinander getrennt. In der Regel wird im Dialysator das Dialysat in einem Dialysatkreislauf im Gegenstrom zum Blutfluss im Blutkreislauf geführt. Über die semipermeable Membran des Dialysators erfolgt der Stoffaustausch zwischen zu reinigendem Blut auf der einen Seite und Dialysat auf der anderen Seite der Membran. Dabei erfolgt der Stofftransport der Urämie-Toxine über die Membran durch Diffusion oder Konvektion. Die Selektivität des Stoffaustausches wird einerseits durch die Eigenschaften der Membran, wie z. B. der Porengröße, bestimmt, andererseits durch die Zusammensetzung des Dialysates. Geeignete Dialysatoren sind im Stand der Technik beschrieben und deren Verwendung ist dem Fachmann bekannt. Üblicherweise werden Kapillardialysatoren eingesetzt. Der Dialysator umfasst bevorzugt eine semipermeable Membran mit einer Größenausschlussgrenze die ausgewählt ist aus dem Bereich von 10.000 bis 25.000 Da, bevorzugt von 14.000 bis 17.000 Da.

Das zu reinigende Blut wird nach der Entnahme und vor oder während oder sowohl vor als auch während des Kontaktes des zu reinigenden Blutes mit dem Dialysator bzw. der semipermeablen Membran des Dialysators einem hochfrequenten elektromagnetischen Feld ausgesetzt. In einer bevorzugten Variante der erfindungsgemäßen Verwendung wird das zu reinigende Blut bei Eintritt in den Dialysator dem hochfrequenten elektromagnetischen Feld ausgesetzt. Dies hat den Vorteil, dass die Lösung der Urämie-Toxine aus der Proteinbindung bereits zu Beginn der Passage durch den Dialysator erfolgt und somit die volle Kapazität des Dialysators für den Stoffaustausch mit dem Dialysat zur Verfügung steht. Wird das zu reinigende Blut während des Kontaktes mit dem Dialysator dem hochfrequenten elektromagnetischen Feld ausgesetzt, so kann das zu reinigende Blut während der gesamten Passage durch den Dialysator dem hochfrequenten elektromagnetischen Feld ausgesetzt sein oder nur für einen Teil der Passage. Es ist auch möglich, dass das zu reinigende Blut an mehreren Stellen der Passage durch den Dialysator einem hochfrequenten elektromagnetischen Feld ausgesetzt wird.

Zur Lösung der Bindung zwischen Plasmaprotein und Urämie-Toxin wird erfindungsgemäß ein hochfrequentes elektromagnetisches Feld verwendet. Dabei wird das hochfrequente elektromagnetische Feld eine Frequenz aufweisen von 100 kHz bis 1 GHz, bevorzugt von 0,5 MHz bis 100 MHz, besonders bevorzugt von 1 MHz bis 30 MHz, ganz besonders bevorzugt von 1 MHz bis 20 MHz. Das zu reinigende Blut kann dabei einem hochfrequenten elektromagnetischen Feld ausgesetzt werden, welches im Wesentlichen über die Zeit eine konstante Frequenz aufweist. Alternativ kann das hochfrequente elektromagnetische Feld eine variierende Frequenz aufweisen, wobei die Frequenz und/oder Feldstärke in einem regelmäßigen oder einem unregelmäßigen Muster variiert werden kann. In einer beispielhaften Ausführungsform wird das zu reinigende Blut einem hochfrequenten elektromagnetischen Feld ausgesetzt welches mit einer relativ geringen Frequenz beginnt und dessen Frequenz über die Zeit bis zu einer vorher festgelegten maximalen Frequenz gesteigert wird. Alternativ dazu kann das zu reinigende Blut auch einem hochfrequenten elektromagnetischen Feld ausgesetzt werden, welches mit einer hohen Maximalfrequenz beginnt und dessen Frequenz über die Zeit bis zu einer vorher festgelegten Mindestfrequenz verringert wird. Durch die Verwendung eines hochfrequenten elektromagnetischen Feldes mit variierenden Frequenzen wird die Effektivität der Lösung von Bindungen zwischen Urämie-Toxin und Plasmaprotein verbessert.

Um eine effektive Aufhebung der Bindung zwischen Urämie-Toxin und Plasmaprotein zu erreichen, ist es von Vorteil, das hochfrequente elektromagnetische Feld auf das zu reinigende Blut/Plasma für eine bestimmte Zeitspanne einwirken zu lassen, so dass es zur Schwingungsanregung von Atomen der beteiligten Moleküle bzw. der Moleküle insgesamt kommen kann. Dazu kann das zu reinigende Blut erfindungsgemäß dem hochfrequenten elektromagnetischen Feld mindestens für eine Zeitdauer von 1/10 Sekunden ausgesetzt sein, bevorzugt für eine Zeitdauer von mindestens ½ Sekunden, besonders bevorzugt über eine Zeitdauer von mindestens einer Sekunde.

Um eine möglichst effektive Trennung von Urämie-Toxin und Plasmaprotein zu erreichen kann es von Vorteil sein, hochfrequente elektromagnetische Felder mit einer bestimmten elektrischen oder magnetischen Feldstärke zu verwenden. Es kann beispielsweise ein hochfrequentes elektromagnetisches Feld zum Einsatz kommen, welches eine elektrische Feldstärke von ≤ 100 V/m, insbesondere von 0,001 bis 100 V/m, bevorzugt von 0,01 bis 10 V/m, besonders bevorzugt von 0,1 bis 10 V/m aufweist. Es kann beispielsweise ein hochfrequentes elektromagnetisches Feld verwendet werden, welches eine magnetische Flussdichte aufweist von ≤ 100 mTesla, bevorzugt von 0,001 bis 100 mTesla, besonders bevorzugt von 0,01 bis 10 mTesla, insbesondere von 0,01 bis 2 mTesla.

Dem Fachmann sind Mittel und Verfahren zur Erzeugung geeigneter hochfrequenter, elektromagnetischer Felder bekannt. Das hochfrequente, elektromagnetische Feld im erfindungsgemäßen Verfahren kann beispielsweise mittels einer Hochfrequenzspule, einer Hochfrequenzelektrode und/oder einem Hochfrequenzkondensator erzeugt werden.

Die vorliegende Erfindung bezieht sich auch auf ein Dialysegerät zur Durchführung der erfindungsgemäßen Verwendung. Ein Dialysegerät umfasst in der Regel einen Dialysatkreislauf, einen Blutkreislauf und einen Dialysator für den Stoffaustausch zwischen zu reinigendem Blut des Blutkreislaufs und Dialysat des Dialysatkreislaufs.

Im Dialystkreislauf wird das Dialysat bewegt, gegen welches das zu reinigende Blut dialysiert werden soll. Dabei wird unter dem Begriff "Dialysatkreislauf" eine Leitungsanordnung verstanden, in der das Dialysat aus einem Reservoir z. B. durch eine Pumpe derart gerichtet durch den Dialysator bewegt werden kann, so dass die Dialysatflüssigkeit den Dialysator im Gegenstrom zum zu reinigenden Blut auf der dem Blut abgewandten Seite der Membran des Dialysators geführt wird. Nach der Passage durch den Dialysator kann das Dialysat abgeführt und ggf. in einem weiteren Behälter aufgefangen und gesammelt werden. Alternativ dazu kann das Dialysat dem Dialysatkreislauf für eine weitere Passage des Dialysators zugeführt werden.

Im Blutkreislauf wird das zu reinigende Blut bewegt. Dabei wird unter dem Begriff "Blutkreislauf" eine Leitungsanordnung verstanden, in der das zu reinigende Blut dem Patienten entnommen wird und z. B. durch eine Pumpe derart gerichtet durch den Dialysator bewegt werden kann, so dass das zu reinigende Blut den Dialysator im Gegenstrom zum Dialysat auf der dem Dialysat abgewandten Seite der semipermeablen Membran des Dialysators geführt wird. Nach der Passage durch den Dialysator wird das gereinigte Blut dem Patienten wieder zurückgeführt.

Das erfindungsgemäße Dialysegerät weist zusätzlich Mittel auf zur Durchführung der erfindungsgemäßen Verwendung, insbesondere Mittel zur Erzeugung eines hochfrequenten elektromagnetischen Feldes. Dem Fachmann sind Mittel und Verfahren zur Erzeugung geeigneter hochfrequenter, elektromagnetischer Felder bekannt. Das erfindungsgemäße Dialysegerät kann zur Erzeugung eines hochfrequenten, elektromagnetischen Feldes beispielsweise eine Hochfrequenzspule, eine Hochfrequenzelektrode und/oder einen Hochfrequenzkondensator aufweisen.

Die Mittel zur Erzeugung eines hochfrequenten, elektromagnetischen Feldes können derart ausgestaltet und im bzw. am Blutkreislauf angeordnet sein, dass das zu reinigende Blut dem hochfrequenten elektromagnetischen Feld ausgesetzt werden kann vor, während oder sowohl vor als auch während des Kontaktes des zu reinigenden Blutes mit dem Dialysator bzw. mit der semipermeablen Membran des Dialysators.

Das erfindungsgemäße Dialysegerät kann zusätzlich eine Regel- und/oder Steuereinheit aufweisen. Diese Regel- und/oder Steuereinheit kann derart ausgebildet sein, dass darüber Parameter des hochfrequenten elektromagnetischen Feldes regel- und/oder steuerbar sind. Dabei kann es sich um solche Parameter wie z. B. die Frequenz, die elektrische Feldstärke, die magnetische Flussdichte und/oder die Dauer des hochfrequenten elektromagnetischen Feldes handeln. Dazu kann die Regel- und/oder Steuereinheit eine Eingabeeinheit, eine Recheneinheit und ggf. eine Speichereinheit umfassen über die Parameter des hochfrequenten elektromagnetischen Feldes durch einen Benutzer des Dialysegerätes regel- und/oder steuerbar sind. In einer bevorzugten Ausführungsform ist die Regel- und/oder Steuereinheit derart ausgebildet, dass sich darüber auch Parameter des Dialysekreislaufs und/oder des Blutkreislaufes, wie z. B. die Flussgeschwindigkeit des zureinigenden Blutes und/oder der Dialysatflüssigkeit und/oder des Dialysates, durch einen Benutzer regeln und/oder steuern lassen.

Im Folgenden wird die Erfindung anhand von Ausführungsbeispielen näher erläutert. Figuren:
Figur 1 zeigt eine schematische Darstellung eines erfindungsgemäßen Dialysegerätes.
Figur 2 zeigt die Urämie-Toxin-Menge (rel. Peakflächen) im Filtrat in An- und Abwesenheit eines HF-Feldes (OH-HPA=p-Hydroxyhippursäure; PAA=Phenylessigsäure; IDS=Indoxylsulfat).
Figur 3 zeigt die Protein-Konzentration im Filtrat in An- und Abwesenheit eines HF-Feldes bei zwei baugleichen Modulen (kein signifikanter Unterschied)

### Beispiele:

### Beispiel 1: Beschreibung eines erfindungsgemäßen Dialysegerätes

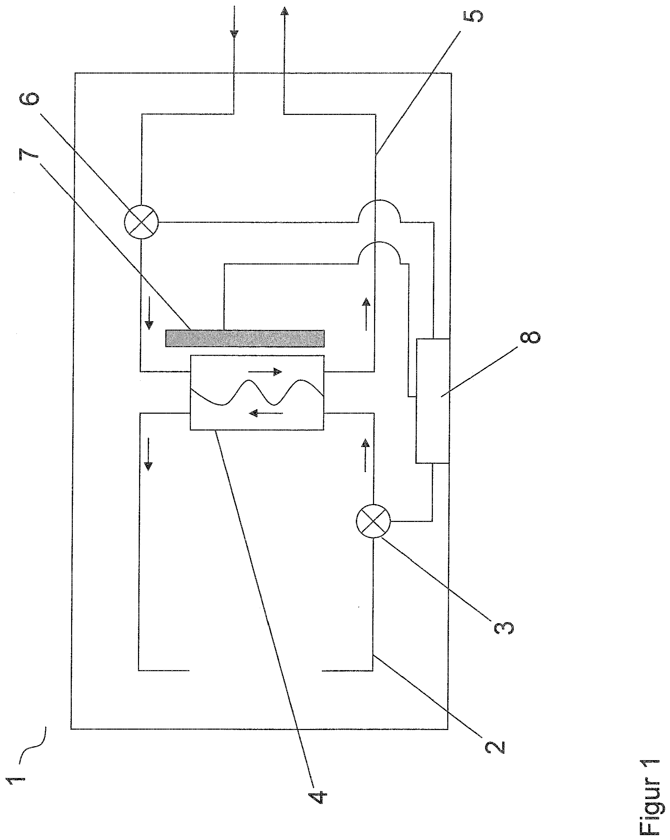

In Figur 1 ist ein erfindungsgemäßes Dialysegerät 1 schematisch dargestellt, welches sich zur Durchführung der erfindungsgemäßen Verwendung eignet. Das Dialysegerät 1 weist einen Dialysatkreislauf 2, einen Blutkreislauf 5 und einen Dialysator 4 auf, die derart miteinander verbunden vorliegen, dass im Dialysator 4 zu reinigendes Blut im Blutkreislauf 5 und Dialysat im Dialysatkreislauf 2 im Gegenstrom auf unterschiedlichen Seiten der semipermeablen Membran aneinander vorbeigeführt werden können, so dass ein Stoffaustausch zwischen Blut und Dialysat über die semipermeable Membran des Dialysators 4 ermöglicht ist. Für den gerichteten Transport von Blut im Blutkreislauf 5 kann eine Pumpe 6 vorgesehen sein. Für den gerichteten Transport von Dialysat im Dialysatkreislauf kann eine Dialysat-pumpe 3 vorgesehen sein. Der Dialysator 4 kann beispielsweise als Kapillardialysator mit einer semipermeablen Membran mit einem Größenausschluss von 10.000 Da bis 20.000 Da ausgestaltet sein. Grundsätzlich kann der Aufbau des erfindungsgemäßen Dialysegerätes 1 aufgrund bekannter, herkömmlicher Dialysetechnologie erfolgen, wobei grundsätzlich alle bekannten Dialysegeräte bzw. Dialysevorrichtungen als Basis verwendet werden können. Zusätzlich weist das Dialysegerät 1 Mittel 7 zur Erzeugung eines hochfrequenten elektromagnetischen Feldes auf. Solche Mittel 7 können beispielsweise durch eine Hochfrequenzspule, eine Hochfrequenzelektrode und/oder einen Hochfrequenzkondensator verkörpert sein. Das erfindungsgemäße Dialysegerät 1 kann zusätzlich eine Regel- und/oder Steuereinheit 8 aufweisen. Diese Regel- und/oder Steuereinheit 8 kann derart mit den Mitteln 7 verbunden und ausgebildet sein, dass darüber Parameter der Mittel 7 zur Erzeugung eines hochfrequenten elektromagnetischen Feldes regel- und/oder steuerbar sind. Dabei kann es sich um solche Parameter wie z. B. die elektrische Frequenz, die elektrische Feldstärke, die magnetische Flussdichte und/oder die Dauer des hochfrequenten, elektromagnetischen Feldes handeln. Dazu kann die Regel- und/oder Steuereinheit 8 eine Eingabeeinheit, eine Recheneinheit und eine Speichereinheit umfassen über der Benutzer des Dialysegerätes 1 die Parameter des hochfrequenten elektromagnetischen Feldes regeln und/oder steuern kann. In einer bevorzugten Ausführungsform ist die Regel- und/oder Steuereinheit 8 derart ausgebildet, dass sich darüber auch Parameter des Dialysekreislaufs 2 und/oder des Blutkreislaufes 5, wie z. B. die Flussgeschwindigkeiten des zu reinigenden Blutes und/oder des Dialysates, durch einen Benutzer regeln und/oder steuern lassen.

### Beispiel 2 Nachweis des Effektes

In *in*-*vitro*-Versuchsreihen wurde der Einfluss hochfrequenter elektromagnetischer Felder auf den proteingebundenen Anteil der Urämietoxine untersucht. Hierzu wurde ein Dialysemodul erstellt, indem konventionelle Hämofiltrations-Kapillaren mit Hilfe von Silikon als Schlaufen in einen Spritzenaufnahmestutzen eingegossen wurden. In das betreffende Modul wurde eine wässerige Albumin-Lösung in Gegenwart der Urämie-Toxine Phenylessigsäure, p-Hydroxyhippursäure und Indoxylsulfat eingebracht. Mit Hilfe einer Spritzenpumpe wurde diese Lösung mit dem Dialysemodul für 10 min filtriert. Anschließend wurde durch Verwendung einer Hochfrequenz-Elektrode (HF-Elektrode) ein hochfrequentes elektromagnetisches Feld in der Lösung induziert. Das elektromagnetische Feld wird mittels einer Hochfrequenzspannungsquelle über einen Zeitraum von 10 Minuten von 1 bis 20 MHz in 1 MHz-Schritten inkrementiert. In den resultierenden Filtraten wurden die Konzentration der zuvor zum künstlichen Plasma gegebenen Urämie-Toxine Phenylessigsäure, p-Hydroxyhippursäure und Indoxylsulfat bestimmt. Durch Vergleich der Urämie-Toxin-Konzentrationen in den resultierenden Filtraten konnte der Effekt des HF-Feldes auf die Bindung zwischen Proteine und Urämie-Toxine evaluiert werden.

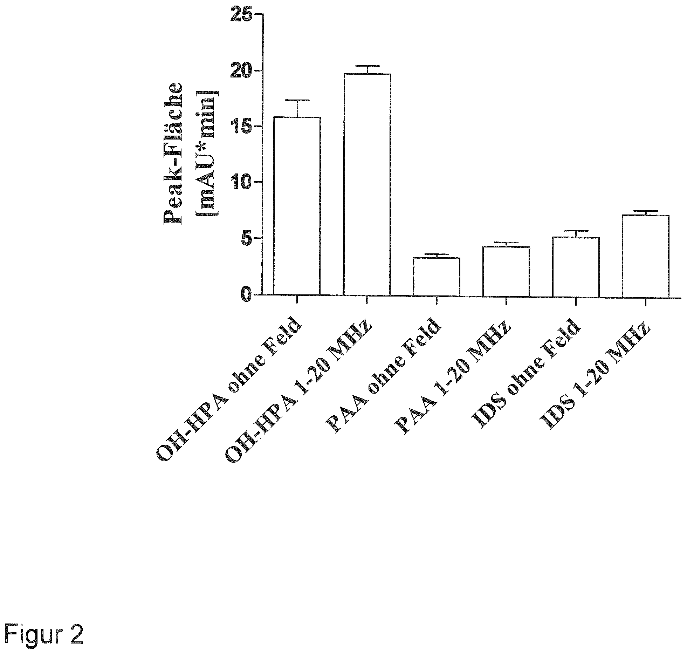

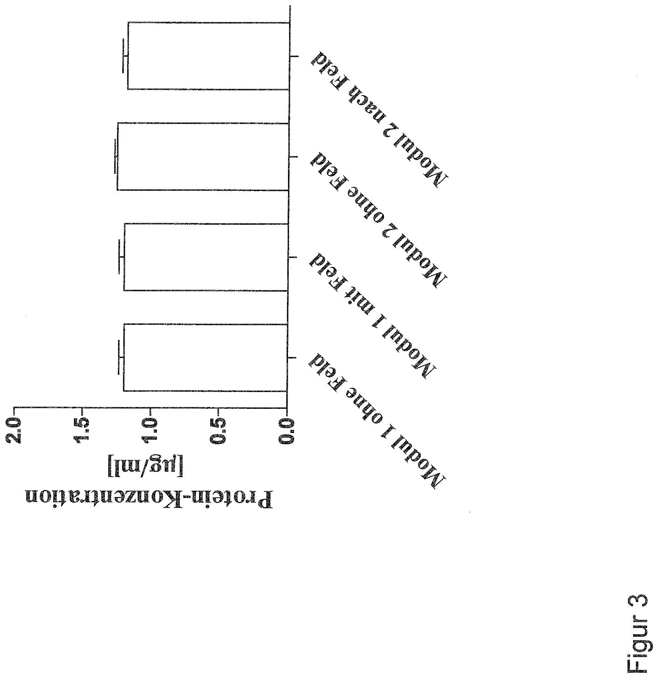

Die quantitative Bestimmung der Urämie-Toxin-Konzentration in den resultierenden Filtraten zeigte, dass hochfrequente elektromagnetische Felder die Filtrationsraten proteingebundener Urämie-Toxine signifikant erhöhen (Figur 2). Um zu überprüfen, ob hochfrequente elektromagnetische Felder die Dialysemembranen beschädigen, wurde die Proteinkonzentration im Filtrat mittels Proteinfärbung nach Bradford bestimmt. Die Ergebnisse zeigen, dass keine signifikanten Änderungen der Proteinkonzentration in Dialysemodulen ohne und unter Einfluss von hochfrequenten elektromagnetischen Feldern nachweisbar sind (Figur 3). Eine makroskopische Beschädigung der Membran ist aufgrund dieser Daten auszuschließen.

### Bezugszeichenliste:

- 1: Dialysegerät
- 2: Dialysatkreislauf
- 3: Dialysatpumpe
- 4: Dialysator
- 5: Blutkreislauf
- 6: Pumpe
- 7: Mittel zur Erzeugung eines hochfrequenten elektromagnetischen Feldes
- 8: Regel- und/oder Steuereinheit

## Patentansprüche

1. Dialysegerät umfassend einen Dialysatkreislauf, einen Blutkreislauf und einen Dialysator, **dadurch gekennzeichnet, dass** das Dialysegerät Mittel aufweist zur Erzeugung eines hochfrequenten elektromagnetischen Feldes mit einer Frequenz von 0,0001 GHz bis 1 GHz und wobei diese Mittel derart angeordnet sind, dass zu reinigendes Blut dem hochfrequenten elektromagnetischen Feld vor und/oder während des Kontaktes mit dem Dialysator aussetzbar ist.

2. Dialysegerät nach Anspruch 1, wobei die Mittel eine Hochfrequenzspule, eine Hochfrequenzelektrode und/oder einen Hochfrequenzkondensator umfassen oder daraus bestehen.

3. Dialysegerät nach einem der Ansprüche 1 oder 2, wobei das Dialysegerät eine Regel- und/oder Steuereinheit umfasst, über die Parameter des hochfrequenten elektromagnetischen Feldes regel- und/oder steuerbar sind.

4. Dialysegerät nach Anspruch 3, wobei die Regel- und/oder Steuereinheit eine Eingabeeinheit, eine Recheneinheit und eine Speichereinheit umfasst über die Parameter des hochfrequenten elektromagnetischen Feldes durch einen Benutzer regel- und/oder steuerbar sind.

5. Dialysegerät nach Anspruch 4, wobei das Dialysegerät derart ausgebildet ist, dass über die Regel- und/oder Steuereinheit auch Parameter des Dialysatkreislaufes und/oder des Blutkreislaufes durch einen Benutzer regel- und/oder steuerbar sind.

6. Dialysegerät nach einem der vorhergehenden Ansprüche, wobei das Dialysegerät derart ausgebildet ist, dass das zu reinigende Blut während der gesamten oder einem Teil der Passage durch den Dialysator dem hochfrequenten elektromagnetischen Feld ausgesetzt ist.

7. Dialysegerät nach einem der vorhergehenden Ansprüche, wobei das Mittel ein hochfrequentes elektromagnetisches Feld mit einer Frequenz von 0,5 MHz bis 100 MHz erzeugt, besonders bevorzugt von 1 MHz bis 30 MHz.

8. Dialysegerät nach einem der vorhergehenden Ansprüche, wobei das Mittel ein hochfrequentes elektromagnetisches Feld mit im Wesentlichen über die Zeit konstanter Frequenz oder einer regelmäßig oder unregelmäßig variierenden Frequenz erzeugt.

9. Dialysegerät nach einem der vorhergehenden Ansprüche, wobei das Mittel derart angeordnet und ausgebildet ist, dass das zu reinigende Blut dem hochfrequenten elektromagnetischen Feld mindestens über eine Zeitdauer von 1/10 Sekunden aussetzbar ist, bevorzugt von mindestens ½ Sekunden, besonders bevorzugt von mindestens 1 Sekunde.

10. Dialysegerät nach einem der vorhergehenden Ansprüche, wobei das Mittel ein hochfrequentes elektromagnetisches Feld mit einer elektrischen Feldstärke von ≤ 100 V/m erzeugt.

11. Dialysegerät nach einem der Ansprüche 1 bis 9, wobei das Mittel ein hochfrequentes elektromagnetisches Feld mit einer elektrischen Feldstärke von ≤ 100 mTesla erzeugt.

12. Dialysegerät nach einem der vorhergehenden Ansprüche, wobei der Dialysator eine semipermeable Membran aufweist und das zu reinigende Blut dem hochfrequenten elektromagnetischen Feld vor und/oder während des Kontaktes des Blutes mit der semipermeablen Membran des Dialysators aussetzbar ist.

## Claims

1. A dialysis machine comprising a dialysate flow system, a blood flow system and a dialyzer, **characterized in that** the dialysis machine has means for generating a high-frequency electromagnetic field having a frequency from 0.0001 GHz to 1 GHz and wherein said means are arranged in such a manner that blood to be cleaned can be exposed to the high-frequency electromagnetic field prior to and/or during contact with the dialyzer.

2. The dialysis machine according to Claim 1, wherein the means comprises or consists of a high-frequency coil, a high-frequency electrode and/or a high-frequency capacitor.

3. The dialysis machine according to any one of Claims 1 or 2, wherein the dialysis machine comprises a regulating and/or control unit by means of which parameters of the high-frequency electromagnetic field can be regulated and/or controlled.

4. The dialysis machine according to Claim 3, wherein the regulating and/or control unit comprises an input unit, a computing unit and a memory unit, by means of which a user can regulate and/or control parameters of the high-frequency electromagnetic field.

5. The dialysis machine according to Claim 4, wherein the dialysis machine is designed such that a user can also regulate and/or control parameters of the dialysate flow system and/or the blood flow system by means of the regulating and/or control unit.

6. The dialysis machine according to any one of the preceding claims, wherein the dialysis machine is designed such that the blood to be cleaned is exposed to the high-frequency electromagnetic field during the entire passage through the dialyzer or part of said passage.

7. The dialysis machine according to any one of the preceding claims, wherein the means generates a high-frequency electromagnetic field having a frequency from 0.5 MHz to 100 MHz, particularly preferably from 1 MHz to 30 MHz.

8. The dialysis machine according to any one of the preceding claims, wherein the means generates a high-frequency electromagnetic field whose frequency is substantially constant over time or varies in a regular or irregular manner.

9. The dialysis machine according to any one of the preceding claims, wherein the means is arranged and designed such that the blood to be cleaned can be exposed to the high-frequency electromagnetic field for a time of at least 1/10 seconds, preferably of at least 1/2 seconds, particularly preferably of at least 1 second.

10. The dialysis machine according to any one of the preceding claims, wherein the means generates a high-frequency electromagnetic field having an electric field strength of ≤100 V/m.

11. The dialysis machine according to any one of Claims 1 to 9, wherein the means generates a high-frequency electromagnetic field having an electric field strength of ≤100 mTesla.

12. The dialysis machine according to any one of the preceding claims, wherein the dialyzer has a semipermeable membrane and the blood to be cleaned can be exposed to the high-frequency electromagnetic field before and/or while said blood is in contact with the semipermeable membrane of the dialyzer.

## Revendications

1. Appareil de dialyse comprenant un circuit de dialysat, un circuit sanguin et un dialyseur, **caractérisé en ce que** l'appareil de dialyse comprend des dispositifs servant à générer un champ électromagnétique haute fréquence d'une fréquence de 0,0001 GHz à 1 GHz, lesdits dispositifs étant disposés de façon à pouvoir exposer le sang à épurer au champ électromagnétique haute fréquence avant et/ou pendant le contact avec le dialyseur.

2. Appareil de dialyse selon la revendication 1, dans lequel les dispositifs comprennent ou sont constitués d'une bobine haute fréquence, d'une électrode haute fréquence et/ou d'un condensateur haute fréquence.

3. Appareil de dialyse selon l'une des revendications 1 ou 2, dans lequel l'appareil de dialyse comprend une unité de réglage et/ou de commande via laquelle les paramètres du champ électromagnétique haute fréquence peuvent être réglés ou commandés.

4. Appareil de dialyse selon la revendication 3, dans lequel l'unité de réglage et/ou de commande comprend une unité de saisie, une unité de calcul et une unité de stockage via lesquelles les paramètres du champ électromagnétique haute fréquence peuvent être réglés ou commandés par un utilisateur.

5. Appareil de dialyse selon la revendication 4, dans lequel l'appareil de dialyse est conçu de telle sorte que les paramètres du circuit de dialysat et/ou du circuit sanguin peuvent être réglés ou commandés via l'unité de réglage et/ou de commande par un utilisateur.

6. Appareil de dialyse selon l'une quelconque des revendications précédentes, dans lequel l'appareil de dialyse est conçu de telle sorte que le sang à épurer, durant tout ou partie de son passage dans le dialyseur, soit exposé au champ électromagnétique haute fréquence.

7. Appareil de dialyse selon l'une quelconque des revendications précédentes, dans lequel le dispositif génère un champ électromagnétique haute fréquence d'une fréquence de 0,5 à 100 MHz, et de préférence de 1 à 30 MHz notamment.

8. Appareil de dialyse selon l'une quelconque des revendications précédentes, dans lequel le dispositif génère un champ électromagnétique haute fréquence avec une fréquence essentiellement constante dans le temps ou une fréquence variant soit de manière régulière, soit de manière irrégulière.

9. Appareil de dialyse selon l'une quelconque des revendications précédentes, dans lequel le dispositif est disposé et conçu de telle sorte que le sang à épurer puisse être exposé au champ électromagnétique haute fréquence pendant une durée de 1/10 de seconde, de préférence pendant au moins ½ seconde, et mieux encore pendant au moins 1 seconde.

10. Appareil de dialyse selon l'une quelconque des revendications précédentes, dans lequel le dispositif produit un champ électromagnétique haute fréquence d'une intensité de champ électrique de ≤ 100 V/m.

11. Appareil de dialyse selon l'une quelconque des revendications 1 à 9, dans lequel le dispositif produit un champ électromagnétique haute fréquence d'une intensité de champ électrique de ≤ 100 mTesla.

12. Appareil de dialyse selon l'une quelconque des revendications précédentes, dans lequel le dialyseur comprend une membrane semi-perméable et le sang à épurer peut être exposé au champ électromagnétique haute fréquence avant et/ou pendant le contact du sang avec la membrane semi-perméable du dialyseur.
